(19) Europäisches Patentamt
European Patent Office
Office européen des brevets

(11) Veröffentlichungsnummer: **0 166 684**
**B1**

(12) # EUROPÄISCHE PATENTSCHRIFT

(45) Veröffentlichungstag der Patentschrift: **20.04.88**

(21) Anmeldenummer: **85810242.9**

(22) Anmeldetag: **23.05.85**

(51) Int. Cl.⁴: **C 07 C 97/26,** C 07 C 39/08, C 07 C 50/34, C 07 C 37/02, C 07 C 46/00, C 09 B 1/503, C 09 B 1/08

(54) **Verfahren zur Herstellung von Hydroxyaromaten.**

(30) Priorität: **29.05.84 CH 2633/84**

(43) Veröffentlichungstag der Anmeldung: **02.01.86 Patentblatt 86/1**

(45) Bekanntmachung des Hinweises auf die Patenterteilung: **20.04.88 Patentblatt 88/16**

(84) Benannte Vertragsstaaten: **BE CH DE FR GB LI**

(56) Entgegenhaltungen:
**FR - A - 2 279 719**
**US - A - 2 487 110**

(73) Patentinhaber: **CIBA-GEIGY AG, Klybeckstrasse 141, CH-4002 Basel (CH)**

(72) Erfinder: **Müller, Rolf, Herrenweg 77, CH-4147 Aesch (CH)**
Erfinder: **Grélat, Maurice, Dr., Habermarkweg 41, CH-4126 Bettingen (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 166 684 B1

**Beschreibung**

Die Erfindung betrifft ein Verfahren zur Herstellung von mindestens eine Hydroxygruppe aufweisenden mono- oder polycyclischen, aromatischen Verbindungen ausgehend von den entsprechenden halogenierten Aromaten durch Austausch des oder der Halogenatome gegen die Hydroxygruppe.

Es ist bekannt in Halogenanthrachinonen die Halogenatome ganz oder teilweise durch die Hydroxygruppe zu ersetzen indem man die betreffenden Halogenanthrachinone mit rauchender Schwefelsäure unter Zusatz von Borsäure bei Temperaturen von etwa 140°C behandelt. Dieses Verfahren, welches technisch angewendet wird, besitzt jedoch den Nachteil, dass die Schwefelsäureregeneration sehr erschwert ist, da sich die Schwefelsäure mit der Borsäure teilweise zu glasigen Produkten verbindet; dadurch wird das Verfahren unwirtschaftlich.

Es wurde nun ein neues Verfahren entwickelt, das den genannten Nachteil ausschaltet und die Regeneration der Schwefelsäure wesentlich erleichtert, und das zudem bei niedrigeren Temperaturen durchgeführt werden kann.

Dieses neue erfindungsgemässe Verfahren besteht darin, dass man halogenierte mono- oder polycyclische Aromaten mit konzentrierter Schwefelsäure unter Zusatz eines Aldehyds bei erhöhter Temperatur behandelt und so die Halogenatome ganz oder teilweise durch die Hydroxygruppe ersetzt.

Unter konzentrierter Schwefelsäure ist vor allem eine solche zu verstehen, deren Konzentration 70 bis 100 Gew.-%, insbesondere 98 bis 100 Gew.-% beträgt (Schwefelsäuremonohydrat). Zudem kann die konzentrierte Schwefelsäure noch bis etwa 25 Gew.-% Schwefeltrioxid enthalten (Oleum).

Als Aldehyde können erfindungsgemäss sowohl aliphatische Aldehyde als auch aromatische Aldehyde eingesetzt werden. Bevorzugt verwendet man aliphatische Aldehyde, vorteilhaft solche, die in ihrer mono-molekularen Form 1 bis 4 Kohlenstoffatome enthalten. Von diesen kommen besonders Formaldehyd bzw. seine polymere Modifikation (Paraformaldehyd) oder ein Formaldehyd abgebendes Reagenz wie z.B. Urotropin oder Paraldehyd in Frage; geeignet sind des weiteren z.B. Acetaldehyd, Propionaldehyd, n-Butyraldehyd und iso-Butyraldehyd. Bei den aromatischen Aldehyden werden gute Ergebnisse erzielt mit Benzaldehyd oder p-Chlorbenzaldehyd.

Der Aldehyd wird, bezogen auf die Ausgangsverbindung, den halogenierten mono- oder polycyclischen Aromaten, in Mengen von 0,25 bis 6 Äquivalenten, insbesondere in Mengen von 0,5 bis 2 Äquivalenten eingesetzt.

Die Temperatur, bei welcher das erfindungsgemässe Verfahren durchgeführt wird, liegt bevorzugt zwischen 50 und 200°C, insbesondere zwischen 100 und 150°C. Die Reaktionszeit beträgt je nach Ausgangsmaterial und Reaktionstemperatur üblicherweise ca. 2 bis 10 Stunden.

Das erfindungsgemässe Verfahren, das bevorzugt unter wasserfreien Bedingungen durchgeführt wird, (100%ige Schwefelsäure als Reaktionsmedium und Arbeiten unter Inertgasatmosphäre), eignet sich insbesondere zur Herstellung von Hydroxyanthrachinonen ausgehend von den entsprechenden Halogenanthrachinonen oder deren Derivaten. Derartige Verbindungen entsprechen z.B. der Formel

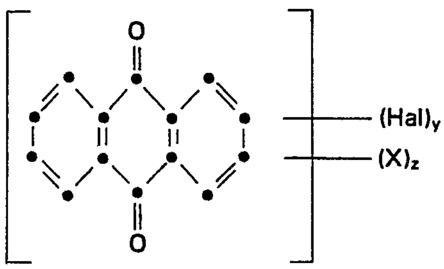

worin bedeuten:

Hal ein Halogenatom, vor allem Cl oder Br;

X Wasserstoff, $NH_2$, Acylamino, vor allem Acetylamino oder OH, und y und z je die Zahlen 1 oder 2.

In diesem Zusammenhang sind als Ausgangsmaterialien beispielsweise erwähnt:

1,5-Diacetylamino-4,8-dibrom-anthrachinon, 1-Amino-2,4-dichloranthrachinon, 1-Amino-2,4-dibromanthrachinon und 1-Chlor-4-hydroxy-anthrachinon.

Des weiteren eignet sich das erfindungsgemässe Verfahren zur Herstellung von z.B. 1,4-Dihydroxybenzoyl (Hydrochinon) aus p-Chlorphenol und von 2,3-Dihydroxynaphthochinon aus 2,3-Dichlornaphthochinon.

Durch das erfindungsgemässe Verfahren werden in Abhängigkeit von vor allem der Temperatur und der Aldehydmenge alle, oder nur ein Teil der Halogenatome durch die Hydroxygruppe ausgetauscht.

Die erhaltenen Hydroxyverbindungen sind wertvolle Zwischenprodukte für die Herstellung von Farbstoffen, vor allem Dispersionsfarbstoffen und Küpenfarbstoffen. Sie werden nach dem erfindungsgemässen Verfahren im allgemeinen in hoher Ausbeute (über 90%, je nach Edukt) erhalten.

Die folgenden Beispiele veranschaulichen die Erfindung. Teile (T) bedeuten Gewichtsteile und Prozente Gewichtsprozente. Die dünnschichtchromatographische Analyse erfolgt auf Kieselgel-Fertigplatten mit UV Indikator.

*Beispiel 1*

184 T Schwefelsäuremonohydrat werden in einem Rührgefäss vorgelegt und unter Rühren mit 4,8 T (1 Äquiv.) Paraformaldehyd während ca. 15 Minuten versetzt. Man rührt weiter bis eine Lösung vorliegt. Hierauf wird auf 60 bis 65°C erwärmt und mit 46,5 T 1-Amino-2,4-dichloranthrachinon (98%) versetzt. Innert 1 Stunde wird auf 110°C erwärmt und 3 Stunden bei dieser Temperatur gehalten. Dann kühlt man auf 100°C und versetzt mit 870 T Wasser. Die erhaltene Suspension wird auf 90 bis 95°C geheizt und 2 Stunden bei dieser Temperatur gerührt; dann wird abgekühlt und filtriert. Der Filterkuchen wird mit heissem Wasser gewaschen und das Produkt im Vakuum bei 80 bis 90°C getrocknet. Man erhält 43,3 T (95% Ausbeute) 1-Amino-2-chlor-4-hydroxyanthrachinon, $R_f$ = 0,29 (Laufmittel: konz. Ameisensäure gesättigt mit 1-Bromnaphthalin).

*Beispiel 2*

184 T Schwefelsäuremonohydrat werden in einem Rührgefäss vorgelegt und unter Rühren 4,2 T (0,8 Äquiv.) Paraformaldehyd während ca. 15 Minuten zugegeben. Man rührt, bis eine klare Lösung vorliegt. Hierauf wird auf 60 bis 65°C erwärmt und mit 59,5 T 1-Amino-2,4-dibromanthrachinon (Reinheitsgrad 96%) versetzt.

Anschliessend erwärmt man das Reaktionsgemisch innerhalb von einer Stunde auf 110°C und rührt bei dieser Temperatur weitere 4 Stunden. Während der Reaktion leitet man Stickstoff durch das Reaktionsgemisch. Anschliessend lässt man auf 60°C abkühlen und tropft 120 T Wasser zu. Die erhaltene Suspension wird filtriert. Der Filterrückstand mit heissem Wasser neutral gewaschen und das Produkt im Vakuum bei 80 bis 90°C getrocknet.

Man erhält 48,5 T (93% Ausbeute d.Th.) 1-Amino-2-brom-4-hydroxyanthrachinon, $R_f$ = 0,27 (Laufmittel: konz. Ameisensäure gesättigt mit 1-Bromnaphthalin).

*Beispiel 3*

184 T Schwefelsäuremonohydrat werden in einem Rührgefäss vorgelegt und unter Rühren mit 43,8 T (2 Äquiv.) 4-Chlorbenzoaldehyd während ca. 15 Minuten versetzt.

Hierauf wird das Reaktionsgemisch auf 60 bis 65°C erwärmt und mit 46,5 T 1-Amino-2,4-dichloranthrachinon (98%) versetzt. Dann erwärmt man innerhalb einer Stunde auf 140°C und hält das Reaktionsgemisch 6 Stunden bei dieser Temperatur. Dann kühlt man das Gemisch auf 100°C ab und versetzt mit 870 T Wasser. Die erhaltene Suspension wird auf 90 bis 95°C erhitzt und 2 Stunden bei dieser Temperatur gerührt, dann wird abgekühlt und filtriert. Der Filterrückstand wird mit heissem Wasser gewaschen und das Produkt im Vakuum bei 80 bis 90°C getrocknet. Das Rohprodukt besteht im wesentlichen aus 1-Amino-2-chlor-4-hydroxyanthrachinon, $R_f$ = 0,29 (Laufmittel: siehe Beispiel 1).

Unter den gleichen Reaktionsbedingungen gelangt man ebenfalls zum 1-Amino-2-chlor-4-hydroxyanthrachinon, wenn man anstelle des 4-Chorbenzaldehyds die gleiche Menge an Benzaldehyd verwendet.

*Beispiel 4*

18,4 T Schwefelsäuremonohydrat werden in einem Rührgefäss vorgelegt und unter Rühren mit 0,48 T Paraformaldehyd während ca. 15 Minuten versetzt. Man rührt bis eine Lösung vorliegt, erwärmt diese dann auf 60 bis 65°C und trägt 4,3 T 1-Chlor--4-hydroxyanthrachinon ein. Innerhalb von einer Stunde erwärmt man das Reaktionsgemisch auf 140°C und lässt während 6 Stunden bei dieser Temperatur reagieren. Dann giesst man das Reaktionsgemisch in 100 T Wasser, filtriert das ausgefallene Produkt ab und wäscht den Filterkuchen neutral. Anschliessend trocknet man das auf diese Weise hergestellte 1,4-Dihydroxyanthrachinon (Chinizarin) bei 80 bis 90°C im Vakuum, $R_f$ = 0,91 (Laufmittel: Toluol gesättigt mit konz. Ameisensäure).

*Beispiel 5*

184 T Schwefelsäuremonohydrat und 20,1 T 4-Chlorphenol werden in einem Rührgefäss vorgelegt und unter Rühren 4,7 T Paraformaldehyd bei Raumtemperatur zugegeben. Das Gemisch wird auf 100°C erwärmt und 3 Stunden bei dieser Temperatur gehalten. Dann kühlt man auf 30°C ab und giesst das Reaktionsgemisch in 870 T Wasser. Nach dem Filtrieren erhält man ein stark saures wasserhaltiges Rohprodukt mit einem Anteil an Hydrochinon von ca. 50%, $R_f$ = 0,54 (Laufmittel: Benzol/Essigsäureäthylester 9:1).

**Patentansprüche**

1. Verfahren zur Herstellung von mindestens eine Hydroxygruppe aufweisenden mono- oder polycyclischen aromatischen Verbindungen ausgehend von den entsprechenden halogenierten Armoaten und Austausch des oder der Halogenatome gegen die Hydroxygruppe, dadurch gekennzeichnet, dass man halogenierte mono- oder polycyclische aromatische Verbindungen mit konzentrierter Schwefelsäure unter Zusatz eines Aldehyds bei erhöhter Temperatur behandelt.

2. Verfahren gemäss Anspruch 1, dadurch gekennzeichnet, dass Schwefelsäure in einer Konzentration von 70 bis 100% verwendet wird.

3. Verfahren gemäss Anspruch 1, dadurch gekennzeichnet, dass Schwefelsäure in einer Konzentration von 98 bis 100% verwendet wird.

4. Verfahren gemäss Anspruch 1, dadurch gekennzeichnet, dass als Aldehyd aliphatische Aldehyde verwendet werden, die in ihrer monomolekularen Form 1 bis 4 Kohlenstoffatome enthalten oder Benzaldehyd oder p-Chlorbenzaldehyd.

5. Verfahren gemäss Anspruch 4, dadurch gekennzeichnet, dass Formaldehyd bzw. seine polymere Modifikation oder ein Formaldehyd abgebendes Reagenz verwendet wird.

6. Verfahren gemäss Anspruch 1, dadurch gekennzeichnet, dass bezogen auf die halogenierte mono- oder polycyclische aromatische Verbindung 0,25 bis 6 Äquivalente Aldehyd verwendet werden.

7. Verfahren gemäss Anspruch 6, dadurch gekennzeichnet, dass bezogen auf Halogenaromat 0,5 bis 2 Äquivalente Aldehyd verwendet werden.

8. Verfahren gemäss Anspruch 1, dadurch gekennzeichnet, dass der Austausch bei einer Temperatur von 50 bis 200°C durchgeführt wird.

9. Verfahren gemäss Anspruch 8, dadurch gekennzeichnet, dass der Austausch bei einer Temperatur von 100 bis 150°C durchgeführt wird.

10. Verfahren gemäss Anspruch 1, dadurch gekennzeichnet, dass als halogenierte mono- oder polycyclische aromatische Verbindung Halogenanthrachinone oder deren Derivate oder halogenierte Phenole eingesetzt werden.

11. Die nach dem Verfahren gemäss Anspruch 1 erhaltenen Hydroxyaromaten.

12. Verwendung der gemäss Anspruch 1 erhaltenen mono- oder polycyclischen Hydroxyaromaten zur Herstellung von Dispersions- oder Küpenfarbstoffen.

**Claims**

1. A process for the preparation of a monocyclic

or polycyclic aromatic compound which contains at least one hydroxyl group, starting from the corresponding halogenated aromatic compound and replacing the halogen atom or atoms by the hydroxyl group, which process comprises treating a halogenated monocyclic or polycyclic aromatic compound with concentrated sulfuric acid, with the addition of an aldehyde, at elevated temperature.

2. A process according to Claim 1, wherein sulfuric acid having a concentration of 70 to 100% is used.

3. A process according to Claim 1, wherein sulfuric acid having a concentration of 98 to 100% is used.

4. A process according to Claim 1, wherein the aldehyde employed is an aliphatic aldehyde which, in its monomolecular form, contains 1 to 4 carbon atoms, or is benzaldehyde or p-chlorobenzaldehyde.

5. A process according to Claim 4, wherein formaldehyde in its monomeric or polymeric form, or a formaldehyde donor, is employed.

6. A process according to Claim 1, wherein 0.25 to 6 equivalents of aldehyde are used, based on the halogenated monocyclic or polycyclic aromatic compound.

7. A process according to Claim 6, wherein 0.5 to 2 equivalents of aldehyde are used, based on the halogenated aromatic compound.

8. A process according to Claim 1, wherein the replacement reactions is carried out in the temperature range from 50 to 200°C.

9. A process according to Claim 8, wherein the replacement reaction is carried out in the temperature range from 100 to 150°C.

10. A process according to Claim 1, wherein the halogenated monocyclic or polycyclic aromatic compound employed is a haloanthraquinone or a derivative thereof or a halogenated phenol.

11. An aromatic hydroxy compound obtained by the process according to Claim 1.

12. Use of a monocyclic or polycyclic aromatic hydroxy compound obtained according to Claim 1 for the manufacture of disperse or vat dyes.

**Revendications**

1. Procédé pour la préparation de composés aromatiques mono- ou polycycliques comportant au moins un groupe hydroxyle, à partir des composés aromatiques halogénés correspondants, et par échange du ou des atomes d'halogène contre le groupe hydroxyle, caractérisé par le fait que l'on traite par l'acide sulfurique concentré des composés aromatiques mono- ou polycycliques halogénés, à température élevée, avec addition d'un aldéhyde.

2. Procédé selon la revendication 1, caractérisé par le fait que l'on utilise de l'acide sulfurique d'une concentration de 70 à 100%.

3. Procédé selon la revendication 1, caractérisé par le fait que l'on utilise de l'acide sulfurique d'une concentration de 98 à 100%.

4. Procédé selon la revendication 1, caractérisé par le fait que l'on utilise, en tant qu'aldéhyde, des aldéhydes aliphatiques qui, sous leur forme monomoléculaire, comportent de 1 à 4 atomes de carbone, ou le benzaldéhyde ou le p-chlorobenzaldéhyde.

5. Procédé selon la revendication 4, caractérisé par le fait que l'on utilise le formaldéhyde ou sa forme polymère, ou un réactif donneur de formaldéhyde.

6. Procédé selon la revendication 1, caractérisé par le fait que l'on utilise 0,25 à 6 équivalents d'aldéhyde, par rapport au composé aromatique mono- ou polycyclique halogéné.

7. Procédé selon la revendication 6, caractérisé par le fait que l'on utilise 0,5 à 2 équivalents d'aldéhyde, par rapport au composé aromatique halogéné.

8. Procédé selon la revendication 1, caractérisé par le fait que l'on effectue l'échange à une température de 50 à 200°C.

9. Procédé selon la revendication 8, caractérisé par le fait que l'on effectue l'échange à une température de 100 à 150°C.

10. Procédé selon la revendication 1, caractérisé par le fait que l'on utilise, en tant que composé aromatique mono- ou polycyclique halogéné, des halogéno-anthraquinones ou leurs dérivés, ou des phénols halogénés.

11. Les composés hydroxy-aromatiques obtenus par le procédé selon la revendication 1.

12. Utilisation des produits hydroxy-aromatiques mono- ou polycycliques obtenus selon la revendication 1, pour la préparation de colorants dispersés ou de colorants de cuve.